# EUROPÄISCHE PATENTSCHRIFT

(11) **EP 0 338 229 B1**
(45) Veröffentlichungstag und Bekanntmachung des Hinweises auf die Patenterteilung: **16.06.1993**
(21) Anmeldenummer: 89104016.4
(22) Anmeldetag: 07.03.1989
(51) Int. Cl.: A61K 39/395, C07K 15/06, C07K 3/02

(54) **Präparat mit Antikörperaktivität und breitem Wirkungsspektrum, daraus bestehende oder diese enthaltende Mittel und deren Verwendung zur Behandlung von bakteriell oder toxin-bedingten Erkrankungen und zur Bekämpfung von Protozoen**
Preparation with antibody activity and a large spectrum of action, remedy consisting of or containing it and its use for the treatment of bacterial or toxin-induced diseases and for combatting protozoa
Préparation à activité d'anticorps et large spectre d'action, remède constitué par ou la contenant et son utilisation pour le traitement de maladies bactériennes ou induites par des toxines et pour lutter contre les protozoaires

(30) Priorität: 19.04.1988 DE 3813043; 02.11.1988 EP 88118243; 24.12.1988 EP 88121678
(43) Veröffentlichungstag der Anmeldung: 25.10.1989
(73) Patentinhaber: BIOTEST PHARMA GMBH, D-63303 Dreieich (DE)
(72) Erfinder: Dichtelmüller, Herbert, Dr., D-6231 Sulzbach/Ts. (DE); Lissner, Reinhard, Dipl.Chem.Dr., D-8761 Weilbach (DE); Stephan, Wolfgang, Dipl.Chem.Dr., D-6072 Dreieich (DE); Arndt, Rüdiger, Dr., D-2000 Hamburg-Blankenese (DE)
(74) Vertreter: Beil, Hans Chr., Dr.

(56) Entgegenhaltungen:
- EP-A- 0 173 999
- EP-A- 0 190 099
- US-A- 4 051 235
- CHEMICAL ABSTRACTS, Band 103, 1985, Seite 494, Nr. 86425x, Columbus, Ohio, US; & JP-A-60 75 433 (NATIONAL FEDERATION OF AGRICULTURAL CO-OPERATIVE ASSOC.) 27-04-1985

## Beschreibung

Die Erfindung betrifft ein Immunglobulin-Präparat mit hoher Antikörperaktivität, welches aus Kolostralmilch von nicht immunisierten Säugern erhältlich ist. Die Erfindung betrifft ferner ein dieses Präparat enthaltendes oder daraus bestehendes Mittel und dessen Verwendung zur Behandlung von bakteriell- oder toxin-bedingten Erkrankungen, insbesondere von Diarrhö bei AIDS oder anderen immunologischen Defektzuständen, von Reisediarrhö und toxin-bedingter Säuglingsdiarrhö, von Magen- und Darmulcera sowie von chronischen und akuten Yersinien-Infektionen, und zur Bekämpfung von Protozoen.

Immunglobuline - eine Gruppe hochwirksamer und gleichzeitig sensibler Proteine - werden u.a. zur Prophylaxe und Behandlung bakterieller oder viraler Erkrankungen und gegebenenfalls in Form spezieller Kompositionen gegen bestimmte Toxine eingesetzt. Sie können nach relativ aufwendigen Verfahren aus humanem Plasma oder auf etwas einfachere Weise aus Milch bzw. Kolostralmilch von Säugern gewonnen werden.

Zur Gruppe der aus humanem Plasma erhältlichen Immunglobuline gehören beispielsweise Immunglobulin G, ein zur Prophylaxe und Behandlung verschiedenster Infektionen wirksames Protein.

Die aus Milch bzw. Kolostralmilch gewonnenen bekannten Immunglobulin-haltigen Präparate weisen eine etwas niedrigere Reinheit auf als die aus humanem Plasma erhältlichen. Sie werden vorwiegend im veterinärmedizinischen Bereich zur Behandlung infektionsbedingter Erkrankungen eingesetzt.

Zahlreiche Verfahren zur Gewinnung von Immunglobulinen aus Milch oder Kolostralmilch sind bisher bekannt.

Die US-PS 3 128 230 betrifft ein Verfahren zur Herstellung eines aus Milch erhältlichen Präparates zur Behandlung verschiedener bakterieller Infektionen.

In der US-PS 4 265 924 und der GB-PS 1 573 995 werden Verfahren zur Herstellung von Proteinkonzentraten aus Lactoserum beschrieben, wobei diese Proteine aufgrund einer Hitzebehandlung grösstenteils denaturiert werden.

Schliesslich werden in der DE-OS 28 13 984 sowie in der EP-A 0 046 909, EP-A 0 064 103 US-A-4 051 235 und der JP-A-60 075 433 Kolostralmilchaufbereitungsverfahren beschrieben, wobei Immunglobulin enthaltende Produkte als entzündungshemmende Mittel erhalten werden.

Bei der Herstellung pharmakologisch wirksamer immunglobulinhaltiger Präparate nach den obengenannten Verfahren wird von Milch bzw. Kolostralmilch hyperimmunisierter Säuger, insbesondere Rinder, ausgegangen. Hierbei müssen also, um eine antibakterielle oder antivirale Effizienz gegen bestimmte Krankheitserreger zu erzielen, den Tieren zunächst ein oder mehrere einen Antikörper bzw. eine Gruppe spezifische Antikörper induzierenden Bakterienstämme bzw. deren Antigene injiziert werden.

Die Wirksamkeit der so gewonnenen Präparate entspricht der Art und Anzahl der verabreichten Bakterienstämme.

Auch zur Bereitstellung eines aus Milch erhältlichen Präparates mit einer spezifischen Antikörperaktivität (z.B. gegen bakterielle Antigene) wird zunächst der Säuger mit speziellen Antigenen immunisiert. Die dann gewonnene Milch weist einen hohen Antikörpertiter gegen das spezielle Antigen auf, wobei die Milch als Trägermedium dient.

Der Erfindung liegt die Aufgabe zugrunde, ein Immunglobulinpräparat hoher Reinheit mit einer hohen Antikörperaktivität nach einem einfachen und wirtschaftlichen Verfahren bereitzustellen, welches im humanmedizinischen und auch im veterinärmedizinischen Bereich angewendet werden kann, ohne dass zuvor eine Hyperimmunisierung vorgenommen werden muss, um spezifische Antikörpertiter bereitzustellen.

Diese Aufgabe wird erfindungsgemäss dadurch gelöst, dass ein Immunglobulinpräparat aus Rinderkolostrum der ersten 30, vorzugsweise ersten 10 Stunden post partum, bei dem die Kolostralmilch mit destilliertem Wasser verdünnt und dann pasteurisiert, das Fett abgetrennt und das Casein ausgefällt und abgetrennt wird, durch Sprühtrocknen der so erhaltenen Kolostralmolke bei einer Eingangstemperatur von 145°C und einer Ausgangstemperatur von 65°C erhalten wird.

Dabei wird ein Immunglobulin enthaltendes Präparat hoher Reinheit mit einem Immunglobulin-Gehalt von >80%, bezogen auf den Gesamtproteingehalt, sowie sehr geringen Anteilen an Fett (<0,4%) und Lactose (<0,1%) erhalten, welches eine höhere Antikörperaktivität aufweist als das bisher als bestwirksamstes humanes Immunglobulin-Präparat bekannte "Pentaglobin"®. Aufgrund der extrem niedrigen antikomplementären Aktivität ist dieses erfindungsgemässe Präparat zur oralen Applikation beim Menschen sowie zur intravenösen Verabreichung in der Veterinärmedizin geeignet.

Das beispielsweise nach Reinigungs- und Trennverfahren, wie sie üblicherweise in der Milchverarbeitung angewendet werden, erhaltene Präparat kann entweder als Lösung oder als Pulver in lagerstabiler Form bereitgestellt werden.

Hierzu wird Kolostralmilch z.B. von Rindern der ersten 10 Stunden post partum mit destilliertem Wasser im Verhältnis 1:3 verdünnt. Aus dieser Lösung wird nach Pasteurisierung das Fett abgetrennt, anschliessend Casein ausgefällt und separiert, z.B. durch Filtration. Die so gewonnene Molke wird auf einen gewünschten Gehalt ankonzentriert. Zur Stabilisierung des Produktes kann eine Sterilfiltration erfolgen, wobei eine sterile Lösung des Präparates erhalten wird.

Nach der Stabilisierung mittels Sprühtrocknung wird das Produkt in pulverisierter Form erhalten. Dabei zeigte sich überraschenderweise, dass bei den für die Sprühtrocknung notwendigen Temperaturen (Eingangstemperatur etwa 145°C bis 180°C, Ausgangstemperatur etwa 65-70°C) keine Denaturierung der Proteine erfolgt. Es tritt also unter diesen Bedingungen der Sprühtrocknung keine extreme Aggregatbildung und damit kein Anstieg der antikomplementären Aktivität der Proteine auf, wie dies eigentlich für solche Temperaturen bekannt und zu erwarten war. Auch bei Anwendung dieser Stabilisierungsmethode wird also ein Produkt mit einem unverändert hohen Immunglobulingehalt von >80% bei unverändert niedriger antikomplementärer Aktivität und vollständiger biologischer Aktivität erhalten.

Zusätzlich kann sowohl vor als auch nach der Durchführung des Stabilisierungsschrittes gemäss einer der genannten Methoden eine Fällungsreaktion mit Octansäure durchgeführt werden. Dabei wird eine weitere Anreicherung des Immunglobulingehaltes auf mehr als 90% erzielt, ohne dass die antikomplementäre Aktivität negativ beeinflusst wird.

Ein Immunglobulin enthaltendes Präparat mit hoher Antikörperaktivität kann auch nach anderen, ebenfalls bekannten Verfahren aus Kolostralmilch der ersten 10 Stunden post partum bereitgestellt werden. Dazu kann beispielsweise ein Verfahren gemäss der DE-PS 3 432 718 angewendet werden. Hierzu wird Kolostralmilch nicht immunisierter Rinder zunächst angesäuert und grob filtriert (Caseinentfernung) und anschliessend mit Kochsalzlösung verdünnt. Die so erhaltene Suspension wird tangential filtriert, anschliessend ankonzentriert und dann neutralisiert. Zur Stabilisierung der konzentrierten Molke kann entweder eine Sterilfiltration oder eine Sprühtrocknung, gegebenenfalls auch eine Octansäurefällung zur Erhöhung der Reinheit des Präparates durchgeführt werden.

### Herstellung des Immunglobulin-Präparates

### Beispiel 1

110 kg tiefgefrorene Rinderkolostralmilch der ersten 10 Stunden nach dem Kalben wurden bei 5-10°C aufgetaut und mit destilliertem Wasser im Verhältnis 1:3 verdünnt. Anschliessend wurde die Milch pasteurisiert und nachfolgend das Fett abgetrennt. Dabei wurden 270 kg Magermilch erhalten. Diese wurde mit 1N HCl angesäuert und das ausgefällte Casein über Filtertücher abgetrennt. Der so gewonnene Überstand (186 kg) wurde ultrafiltriert, wobei 38 kg Konzentrat erhalten wurden.

Dieses Konzentrat wurde zur Stabilisierung entweder auf herkömmliche Weise sterilfiltriert oder sprühgetrocknet. Die Sprühtrocknung erfolgt bei einer Eingangstemperatur von 175°C und einer Ausgangstemperatur von 65°C. Hierbei wurde ein trockenes Pulver erhalten, welches in 83%iger Reinheit Immunglobulin enthält, entsprechend einer Gesamtausbeute von 3,8 kg Immunglobulin.

Die Antikörperaktivität dieses Präparates vor Sprühtrocknung entsprach der nach Sprühtrocknung und auch der eines durch Sterilfiltration stabilisierten Präparates.

### Beispiel 2

13 l Rinderkolostralmilch, die in den ersten 5 Stunden nach dem Kalben entnommen und tiefgefroren worden war, wurden aufgetaut. Der pH-Wert betrug 6,27. Durch Ansäuern mit 700 ml 1N HCl wurde dieser auf 4,8 eingestellt. Die so erhaltene Suspension wurde 30 Minuten lang auf 40°C erwärmt und dann über Nacht bei 4°C aufbewahrt. Anschliessend wurde die Suspension zur Abtrennung grober Partikel durch ein Gazefilter aus Polyamidgaze in ein Vorratsgefäss gepumpt und dort mit 0,9%iger Kochsalzlösung auf ein Gesamtvolumen von 26 l verdünnt.

Dann erfolgte die erste Tangentialfiltration der so erhaltenen Suspension durch eine Hohlfaserpatrone mit einer mittleren Porengrösse von 0,4 µm, einer Oberfläche von 3 m² und einem Faserdurchmesser von 1,2 mm durch Diafiltration unter Verwendung von 100 l einer 0,9%igen Kochsalzlösung bei einem Überdruck von 0,2 bis 0,6 bar.

Noch während des Verlaufs der ersten Tangentialfiltration wurde das angefallene Diafiltrat einer zweiten Tangentialfiltration unter Verwendung einer aus drei Hohlfaserpatronen mit jeweils einer Trenngrenze von 10 000 D und einer Oberfläche von 1,4 m² bestehenden Anlage zugeführt. In dieser Anlage wurde das Diafiltrat der ersten Tangentialfiltration zunächst ankonzentriert, sodann unter Verwendung von 100 l einer 0,9%igen Kochsalzlösung diafiltriert und schliesslich auf ein Gesamtvolumen von 25 l ankonzentriert. Das die niedermolekularen Bestandteile enthaltende Filtrat wurde entfernt.

Die erhaltene Immunglobulinlösung wies einen Proteingehalt von 6% (bestimmt durch Biuret-Reaktion) auf und wurde in bekannter Weise sterilfiltriert.

### Beispiel 3

25 l Konzentrat von Rinderkolostrum wurden nach Beispiel 2 hergestellt. Ein Aliquot von 10 l 10%iger Immunglobulinlösung wurde der Sprühtrocknung bei einer Eingangstemperatur von 145°C und einer Ausgangstemperatur von 65°C unterzogen. Erhalten wurden 1 270 g eines weisslichen Immunglobulin-Pulvers.

Antikörperaktivität und antikomplementäre Aktivität entsprachen der vor der Sprühtrocknung sowie der des nach Beispiel 2 hergestellten sterilfiltrierten Präparates.

### Beispiel 4

200 ml einer Immunglobulinlösung, hergestellt nach Beispiel 1, wurden vor dem Stabilisierungsschritt bei pH 4,8 und einer Temperatur von 23°C 5 Stunden mit Octansäure (2,5%) versetzt. Anschliessend wurde der Niederschlag durch Filtration entfernt und der Überstand gegen 0,9%ige NaCl-Lösung dialysiert.

Der Gehalt an Immunglobulin des so erhaltenen Präparates betrug >90%. Die antikomplementäre Aktivität entsprach dem niedrigen Wert einer intravenös verträglichen humanen IgG-Präparation.

### Beispiel 5

Von einem gemäss Beispiel 1 hergestellten (sprühgetrockneten) Immunglobulinpulver wurden 200 ml sterilfiltrierte Lösung mit einem Proteingehalt von 5% hergestellt. Eine solche Lösung (5 g Pulver, eingerührt in H₂O ad 100 ml) weist folgende Zusammensetzung auf (wobei für alle Proteinwerte eine relative Streubreite von ±10% zu berücksichtigen ist):

| | |
|---|---|
| Protein gesamt | 4,2 g/100 ml |
| davon IgG | 3,0 g/100 ml |
| IgA | 0,35 g/100 ml |
| IgM | 0,96 g/100 ml |
| pH-Wert | 4,6 |
| Lactalbumin | 1,0 % CAF |

Diese Lösung wurde mit 2,5% Octansäure bei pH 4,8 und T=23°C während 5 Stunden inkubiert. Der Niederschlag wurde durch Zentrifugieren abgetrennt und der Überstand diafiltriert. Die antikomplementäre Aktivität entsprach der eines humanen IgG-Präparates gleicher Konzentration (5%) für intravenöse Anwendung.

Das nach den beschriebenen oder analogen Verfahren erhältliche Immunglobulinpräparat weist eine höhere Antitoxinaktivität auf als Pentaglobin.

### Neutralisation bakterieller Toxine

Die Neutralisation hämolysierender Bakterientoxine wurde im Hämolyse-Hemmtest (HHT) gegen toxinhaltige Überstände verschiedener Bakterien bestimmt.

### Beispiel 6

Eine Übernachtkultur in Brain Heart Infusion Nährbouillon von Staphylococcus aureus Smith 3 wurde bei 10.000 xg 10 min zentrifugiert und der Überstand sterilfiltriert. Der keimfreie hämolysierende Toxine enthaltende Überstand dieser Kultur wurde für Hämolyse-Hemmtests verwendet.

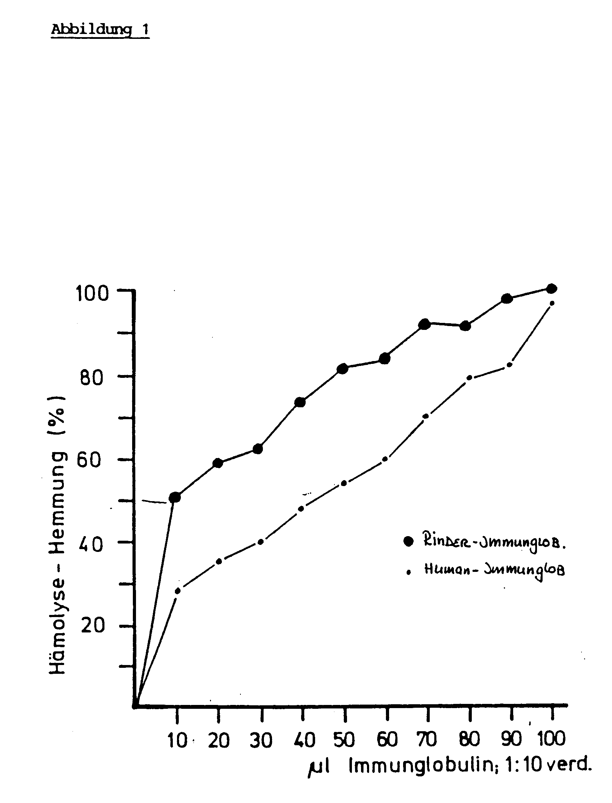

Eine 5%ige Lösung eines nach Beispiel 2 hergestellten Immunglobulinpräparates wurde auf die toxinneutralisierende Wirkung geprüft. Hierzu wurden 0,1 ml gewaschene Humanerythrozyten zu 10 µl Staphylococcus aureus Toxin in 900 bis 990 µl NaCl (0,9%) bei 37°C während 30 min inkubiert, wobei jeweils 10-100 µl Immunglobulinlösung (5%) zugesetzt wurden. Als Vergleichspräparation diente eine 5% kommerziell verfügbare Immunglobulin-Präparation (Pentaglobin). Das Ergebnis zeigt Abbildung 1, wobei als Mass für die Hämolyse bzw. deren Hemmung die Extinktion bei 542 nm zur Messung verwendet wurde.

### Beispiel 7

Es wurde wie in Beispiel 6 angegeben vorgegangen, jedoch wurde hier der toxinhaltige Überstand eines gram-negativen Keimes - Ps. aeruginosa - zur Prüfung auf Toxinneutralisation verwendet. Die Hämolyse wurde wie in Beispiel 6 bzw. 8 im Bereich zwischen 0 und 100 µl der 5% Immunglubulinösung, 1:10 verdünnt in NaCl (0,9%), gemessen.

### Beispiel 8

Für die nachstehend aufgeführte Untersuchung wurde der toxinhaltige Überstand von Staphylococcus aureus verwendet. Die Toxinneutralisation wurde mit einem gemäss Beispiel 1 (sprühgetrocknet) hergestellten Präparat (5%ige Lösung) getestet.
Als Vergleich im HHT diente Pentaglobin in gleicher Konzentration.
Die Hämolyse wurde im Bereich von 0 bis 100 µl Immunglobulinlösung (5%, 1:10 verdünnt in NaCl) gemessen.

Die Ergebnisse aus den Versuchen der Beispiele 6-8 sind in den Abbildungen 1-3 aufgezeigt.

Abbildung 1 zeigt die Neutralisation bakterieller hämolysierender Toxine von Staphylococcus aureus Smith 3 gemäss Beispiel 6 durch Immunglobulin aus Rinderkolostrum, hergestellt nach Beispiel 2, und durch Pentaglobin als Vergleich.

Abbildung 2 zeigt die Neutralisation bakterieller Toxine von Pseudomonas aeruginosa gemäss Beispiel 7 durch Immunglobulin aus Rinderkolostrum, hergestellt nach Beispiel 2.

Abbildung 3 zeigt die Neutralisation bakterieller Toxine von Staphylococcus aureus gemäss Beispiel 8 durch Rinderkolostrum-Immunglobulin, hergestellt nach Beispiel 1 und durch Pentaglobin als Vergleich.

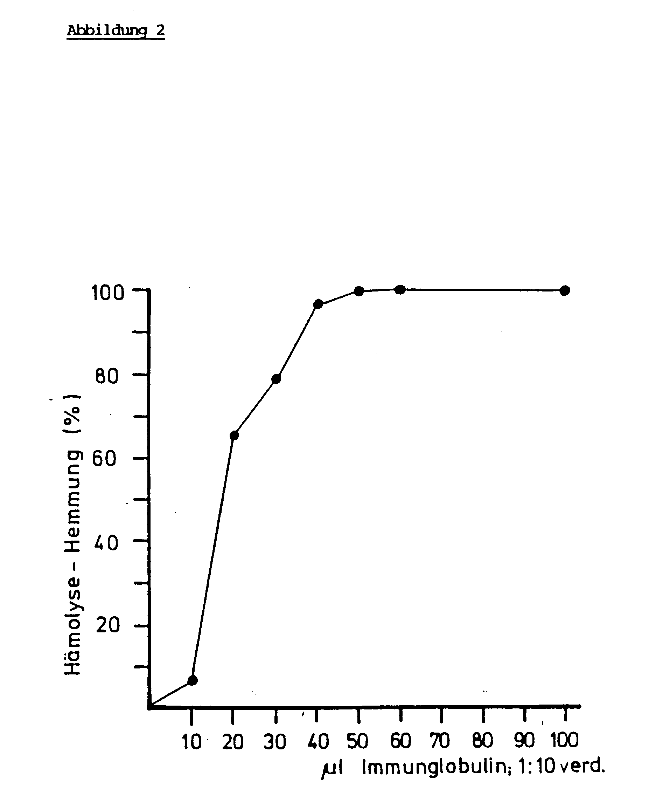

Wie aus den Abbildungen 1-3 ersichtlich ist, bewirkt die aus Rinderkolostrum gewonnene Immunglobulin-Präparation eine effektivere Hemmung der toxinbedingten Hämolyse als das in der Zusammensetzung vergleichbare, aus humanem Plasma gewonnene Pentaglobin.

In Tabelle I sind die Ergebnisse der Versuche gemäss den Beispielen 6-8 zusammengefasst.

**Tabelle I**

| Beispiel | | Konzentration bei 50% Hämolyse | Konzentration bei 80% Hämolyse |
|---|---|---|---|
| 6 | PG (Vergl)* | 50 µl | 93 µl |
| | RG (Erf.)** | 10 µl | 50 µl |
| 7 | RG (Erf.) | 18 µl | 35 µl |
| 8 | PG (Vergl.) | 42 µl | 80 µl |
| | RG (Erf.) | 10 µl | 40 µl |

| | | | |
|---|---|---|---|
| * PG = Pentaglobin | | | |
| ** RG = Immunglobulin aus Rinderkolostrum | | | |

Die Beispiele 6 und 8 belegen, dass für eine 50%ige Hämolyse (Toxinneutralisation) eine um das Dreifache höhere Menge des bekannten Pentaglobins erforderlich ist als für das erfindungsgemässe Präparat. Um eine 80%ige Toxinneutralisation zu erzielen, muss eine doppelt so hohe Menge Pentaglobin eingesetzt werden.

Wie aus Beispiel 7 hervorgeht, bewirkt das erfindungsgemässe Immunglobulin schon bei sehr geringen Konzentrationen (40 µl) eine fast 100%ige Toxinneutralisation, auch gegen Toxine gram-negativer Bakterien.

Eine 100%ige Hämolyse wird in dem betrachteten Konzentrationsbereich (maximal 100 µl einer 5%igen Lösung) für Pentaglobin nicht erreicht. Da das aus Kolostralmilch hergestellte Immunglobulinpräparat im Gegensatz zum Pentaglobin keine 100%ige Reinheit aufweist (80-90%), ist der therapeutische Index anhand vorstehender Ergebnisse für das erfindungsgemässe Antitoxin mindestens doppelt so hoch wie für das bekannte Pentaglobin.

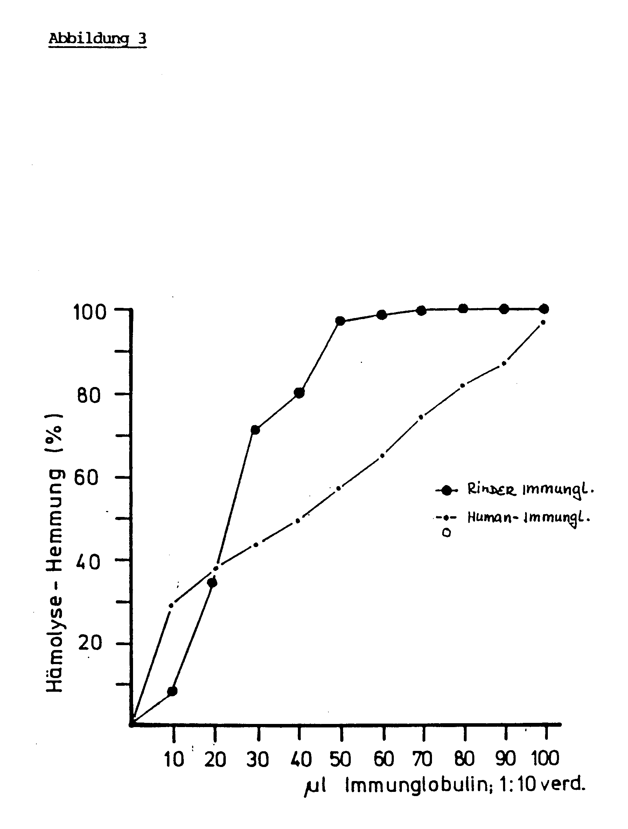

Wie weiterhin aus Tabelle I sowie Abbildung 3 ersichtlich ist, bleibt das gewählte Verfahren zur Bereitstellung des Produktes ohne Einfluss auf dessen Aktivität. Es kann sowohl nach bekannten Methoden der Milchwirtschaft wie auch durch speziellere Aufbereitungsverfahren (z.B. Tangentialfiltration) erhalten werden.

Das erfindungsgemässe Produkt ist im veterinärmedizinischen Bereich intravenös applizierbar und kann am Menschen oral verabreicht werden. Es weist keine anomale Toxizität auf, wie in nachstehenden Beispielen 9 bis 11 dargelegt, und kann sowohl flüssig als auch in fester Form gelagert werden.

### Verträglichkeit - Anomale Toxizität

### Beispiel 9

Je 5 Mäuse erhielten eine wie vorstehend getestete 5%ige Immunglobulinlösung (gemäss den Beispielen 6-8) intraperitoneal appliziert (0,1 ml/Tier). Die Tiere wurden bis 3 Stunden beobachtet und bis 5 Tage nach Gabe gehalten.
- Ergebnis:: Kein Tier verstorben, keine Reaktion sofort, keine Reaktion bis 3 Stunden nach Gabe, keine Veränderungen.
- Dosierung:: 0,29 g/kg (Gewicht Maus: 17 g).

### Beispiel 10

Je 5 Mäuse erhielten 0,5 ml der 5% Lösung, pH 4,6 oral mittels Schlundsonde verabreicht und wurden bis 3 Stunden nach Gabe beobachtet und bis 5 Tage nach Gabe gehalten.
- Ergebnis:: Kein Tier verstorben, keine Reaktion sofort, keine Reaktion bis 5 Stunden, keine Veränderungen.
- Dosierung:: 1,47 g/kg; (Gewicht Maus = 17 g)

### Beispiel 11

Je 3 Mäuse erhielten 1,0 ml einer 10% Suspension, pH 4,6 oral mittels Schlundsonde verabreicht.
- Ergebnis:: Keine Symptome wurden sofort bis 1, nach 3 und nach 24 Stunden beobachtet. Nach 5 Tagen waren alle Tiere unverändert.
- Dosierung:: Die Dosis von 1,0 ml 10% Lösung (0,1 g/Tier) entspricht einer Dosierung von 5,0 g/kg bzw. 350 g/70 kg (Gewicht Maus = 20 g).

Das aus Kolostralmilch erhältliche erfindungsgemässe Immunglobulin-Präparat ist auf sehr einfache ökonomische Weise herstellbar und weist eine unerwartet hohe Antitoxinaktivität gegenüber verschiedensten Bakterientoxinen auf. Es zeichnet sich durch eine ausgezeichnete Verträglichkeit (keine anomale Toxizität) aus und kann in hoher Reinheit (>90%) sowohl in flüssiger Form als auch als Pulver in stabilisierter Form gelagert werden.

### Beispiel 12

Überraschend wurde gefunden, dass die erfindungsgemässen Präparate ausgezeichnet zur Behandlung schwerer Diarrhöen als Folgeerkrankungen von HIV-Infektionen oder von anderen immunologischen Defektzuständen geeignet sind. Bei Verabreichung der erfindungsgemässen Präparate traten innerhalb kürzester Zeit eine erhebliche Reduzierung der Zahl der täglichen Durchfälle bis hin zum kompletten Sistieren der Diarrhö ein, wie die folgende Tabelle II zeigt. Den an AIDS erkrankten Patienten wurden jeweils täglich 10 g des erfindungsgemässen Präparates über einen Zeitraum von 10 Tagen verabreicht.

**Tabelle II**

| Patient | Vor Therapie | Durchfälle pro Tag | | | | Körpergewicht kg | |
|---|---|---|---|---|---|---|---|
| | | Tage nach Therapiebeginn | | | | vor | nach |
| | | 1 | 3 | 5 | 10 | Therapie | |
| (1) | 10 | 5 | 1 | 1 | 1 | 42,0 | n.t. |
| (2) | 6 | 1 | 0* | 0* | 0* | 84,4 | 85,5 |
| (3) | 10 | 5 | 2 | 2 | 1 | 62,3 | 62,8 |

| | | | | | | | |
|---|---|---|---|---|---|---|---|
| *Stuhl normal | | | | | | | |

Die erfindungsgemässen Präparate können allein oder in Kombination mit anderen Diarrhoika oder anderen für die Therapie notwendigen oder geeigneten Mitteln verabreicht werden und führen innerhalb kürzester Zeit zu einer signifikanten Reduzierung der Durchfallhäufigkeit und damit einer wesentlichen Verbesserung des Allgemeinzustandes des Patienten.

### Beispiel 13

Die erfindungsgemässen Präparate eignen sich überraschend auch zur Behandlung sowohl der sog. Reisediarrhö wie der toxin-bedingten Säuglingsdiarrhö.

In Laboruntersuchungen wurde gefunden, dass das erfindungsgemässe Präparat Antikörper unter anderem gegen das 40 KD-Protein des Verotoxin 1 (VT₁) enthält. VT₁ oder Shiga like Toxin 1 ist neben Verotoxin 2 das wichtigste zytotoxische Exotoxin säuglingspathogener E. coli (EPEC) und enterohaemorrhagischer E. coli (EHEC).

Ferner enthält das erfindungsgemässe Präparat Antikörper gegen 30 KD- und 20 KD-Proteine des hitzelabilen Enterotoxins von enterotoxinbildendem E. coli (ETEC), einer Ursache der Reisediarrhö.

Damit enthält das erfindungsgemässe Präparat Antikörper gegen nahezu das gesamte Spektrum der Toxine darmpathogener Coli-Keime (enterotoxinogene, ETEC; säuglingspathogene, EPEC; enterohaemorrhagische, EHEC). Dies ist insbesondere deshalb ein erheblicher Vorteil, weil diese Erkrankungen häufig auftreten und zumindest teilweise die Folge einer Antibiotikatherapie sein können oder die Antibiotikagabe häufig negative Effekte beim Patienten durch Toxinausschüttung bewirkt.

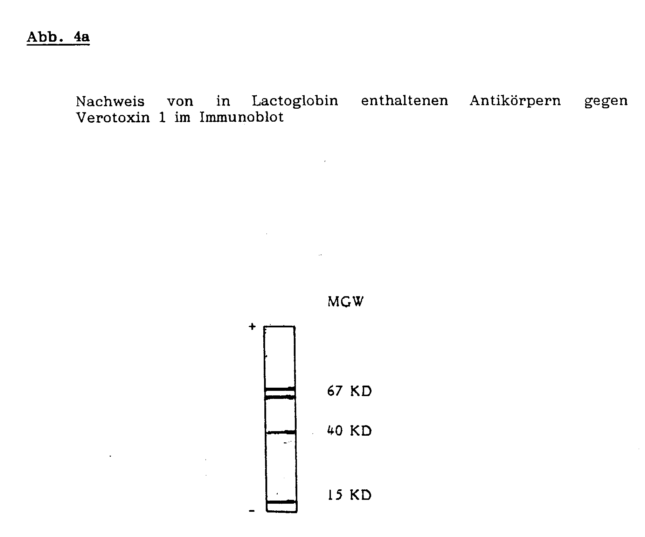

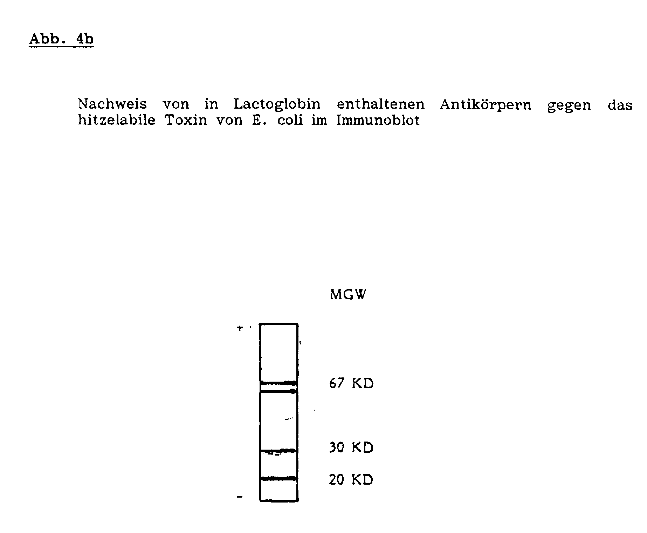

Die Ergebnisse der mittels der Immunoblot-Technik durchgeführten Untersuchungen bei verschiedenen Konzentrationen sind in Abbildung 4 wiedergegeben.

### Beispiel 14

Gastritis assoziierte duodenale und gastrale Ulkuserkrankungen werden bisher mit Wismutsalzen oder Antibiotika behandelt. Die einfachen nebenwirkungsarmen Therapieververfahren (Wismutsalze) haben nur geringe Effektivität, die wirksamen antibiotischen Kombinations-Schemata sind dagegen sicher, für die Routineanwendung wegen der Nebenwirkungen jedoch wenig praktikabel. Es besteht danach ein Bedarf an einfachen nebenwirkungsarmen und zugleich wirksamen Therapeutika. Für diese Erkrankungen sowie für nicht-ulceröse Dyspepsien hat Campylobacter pylori nach heutiger Kenntnis pathogenetische Bedeutung. Charakteristisch für C. pylori sind ein äusseres Membranprotein von 120 KD, ein spezifisches Geisselantigen (Flagellin) von 69 KD und die Urease mit 45 KD.

Es wurde nun überraschend gefunden, dass das erfindungsgemässe Präparat auch Antikörper in hoher Konzentration gegen diese drei Proteine besitzt.

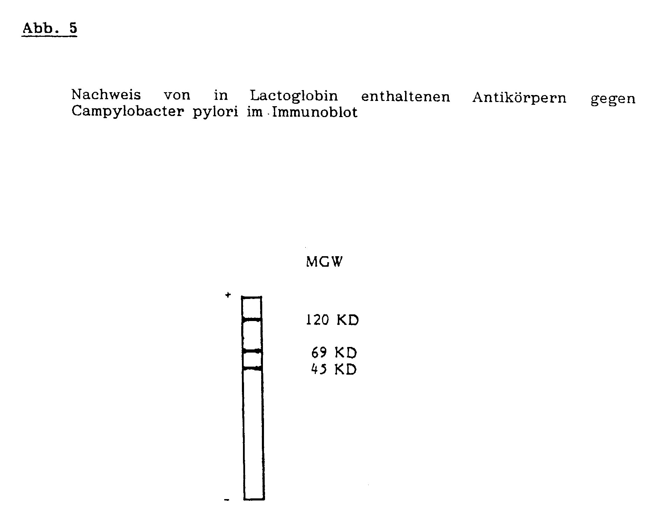

Die Überprüfung erfolgte mittels der Immunoblot-Technik. Das erfindungsgemässe Präparat mit einem Proteingehalt von 4 mg/ml wurde unverdünnt sowie in Verdünnungen von 1:10 und 1:100 überprüft. Die Detection erfolgte durch mit alkalischen Phosphatasen konjugiertem Rinder-IgG. Wie aus den in Abbildung 5 wiedergegebenen Blots ersichtlich ist, reagiert das erfindungsgemässe Präparat bei einer Verdünnung von 1:10 mit allen drei vorgenannten Proteinen und in einer Verdünnung von 1:100 noch deutlich mit dem Flagellin und der Urease.

Darüberhinaus enthält das erfindungsgemässe Präparat noch eine Vielzahl von Antikörpern, die mit der C. pylori-Präparation reagieren.

Damit ist überraschenderweise das erfindungsgemässe Präparat auch für die Behandlung von Infektionen mit Campylobacter pylori ausgezeichnet geeignet.

### Beispiel 15

Yersinieninfektionen führen nicht nur zu einem akut enteritischen Verlauf, sondern vor allem auch zu subakuten und und chronischen Krankheitsformen, die in der Regel mit negativem Kulturbefund einhergehen, wobei sich jedoch immunhistologisch Erreger weiterhin nachweisen lassen. Die wichtigsten Erkrankungen mit positivem IgA-Antikörpernachweis als Zeichen einer bestehenden Infektion sind reaktive Arthritiden, Erythema nodosum, Uveitis, chronische Enteritis u. dgl..

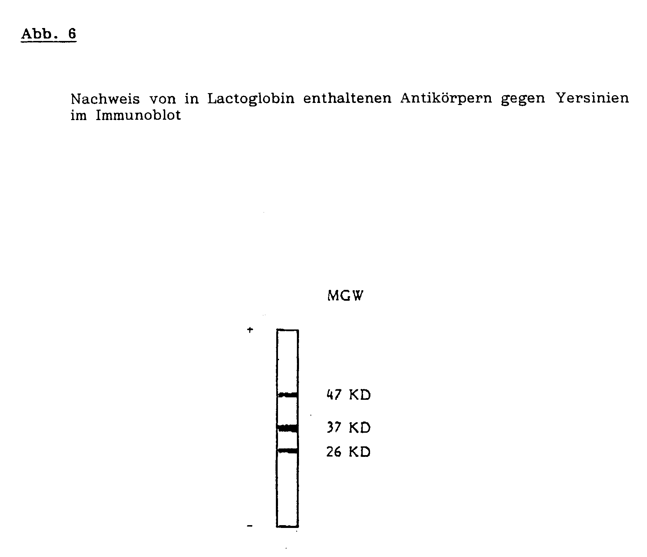

Erfahrungsgemäss enthalten Yersinien speziesunabhängig sogenannte YOP-Antigene (u.a.: YOP 2b mit 47 KD, YOP 3 mit 37 KD, YOP 5 mit 26 KD). Unter Verwendung dieser isolierten Antigene wurde das erfindungsgemässe Präparat in einem sogenannten Westernblot überprüft. Dafür wurde das Präparat unverdünnt (Proteingehalt 4 mg/ml) sowie in Verdünnungen von 1:10 und 1:100 eingesetzt. Das Präparat reagierte, wie aus Abbildung 6 ersichtlich ist, mit allen drei Antigenen bei allen verwendeten Konzentrationen. Die Anfärbung erfolgte dabei mit einem mit alkalischer Phosphatase konjugiertem spezifischen anti-Rind-IgG (H- und L-Ketten) in einer Verdünnung von 1:1500 und 1:3000.

Da die danach erkannten Antigene unabhängig von Serotyp und Spezies der Yersinien sind, ist das erfindungsgemässe Präparat auch ausgezeichnet zur Behandlung von Yersinieninfektionen geeignet.

### Beispiel 16

Die erfindungsgemässen Präparate eignen sich überraschenderweise auch zur Bekämpfung von Protozoen, wie beispielsweise Cryptosporidien, Isospora belli oder Toxoplasma Gondii, für die bisher nicht bei allen Erkrankungsfällen Gegenmittel bekannt waren.

Cryptosporidien führen zu schweren Durchfällen, die bei immundefekten Patienten mit bisher bekannten Mitteln nicht wirksam behandelt werden konnten. Vier Patienten mit schweren Durchfällen, in deren Stuhl Cryptosporidien nachzuweisen waren, wurden an 10 aufeinanderfolgenden Tagen jeweils 10 g des erfindungsgemässen Präparats als Trinklösung verabreicht. Nach Beendigung dieser Therapie war bei keinem dieser Patienten Cryptosporidien-spezifisches Antigen nachweisbar.

## Patentansprüche

1. Immunglobulinpräparat aus Rinderkolostrum der ersten 30, vorzugsweise ersten 10 Stunden post partum, bei dem die Kolostralmilch mit destilliertem Wasser verdünnt und dann pasteurisiert, das Fett abgetrennt und das Casein ausgefällt und abgetrennt wird, erhältlich durch Sprühtrocknen der so erhaltenen Kolostralmolke bei einer Eingangstemperatur von 145°C und einer Ausgangstemperatur von 65°C.

2. Präparat nach Anspruch 1, dadurch gekennzeichnet, daß die Kolostralmolke vor dem Sprühtrocknen ankonzentriert wird.

3. Präparat nach einem der Ansprüche 1 oder 2, dadurch gekennzeichnet, daß es vor dem Sprühtrocknen einer Octansäurebehandlung unterworfen wird.

4. Mittel zur Behandlung von Diarrhö bei AIDS oder anderen immunologischen Defektzuständen, bestehend aus einem Präparat nach einem der Ansprüche 1 bis 3.

5. Mittel zur Behandlung von Diarrhö bei AIDS oder anderen immunologischen Defektzuständen, enthaltend ein Präparat nach einem der Ansprüche 1 bis 3.

6. Verwendung des Präparates nach einem der Ansprüche 1 bis 3 zur Herstellung eines Mittels zur Bekämpfung der Diarrhö bei AIDS oder anderen immunologichen Defektzuständen.

7. Mittel zur Behandlung der Reisediarrhö, bestehend aus einem Präparat nach einem der Ansprüche 1 bis 3.

8. Mittel zur Behandlung der Reisediarrhö, enthaltend ein Präparat nach einem der Ansprüche 1 bis 3.

9. Verwendung eines Präparates nach einem der Ansprüche 1 bis 3 zur Herstellung eines Mittels zur Behandlung der Reisediarrhö.

10. Mittel zur Behandlung der Toxin-bedingten Säuglingsdiarrhö, bestehend aus einem Präparat nach einem der Ansprüche 1 bis 3.

11. Mittel zur Behandlung Toxin-bedingter Säuglingsdiarrhö, enthaltend ein Präparat nach einem der Ansprüche 1 bis 3.

12. Verwendung des Präparates nach einem der Ansprüche 1 bis 3 zur Herstellung eines Mittels zur Behandlung der Toxin-bedingten Säuglingsdiarrhö.

13. Mittel zur Behandlung von Magen- und Darmulcera sowie nicht-ulcerösen Dyspepsien, bestehend aus einem Präparat nach einem der Ansprüche 1 bis 3.

14. Mittel zur Behandlung von Magen- und Darmulcera sowie nicht-ulcerösen Dyspepsien, enthaltend ein Präparat nach einem der Ansprüche 1 bis 3.

15. Verwendung des Präparates nach einem der Ansprüche 1 bis 3 zur Herstellung eines Mittels zur Behandlung von Magen- und Darmulcera sowie nicht-ulcerösen Dyspepsien.

16. Mittel zur Behandlung von chronischen und akuten Yersinien-Infektionen, bestehend aus einem Präparat nach einem der Ansprüche 1 bis 3.

17. Mittel zur Behandlung von chronischen und akuten Yersinien-Infektionen, enthaltend ein Präparat nach einem der Ansprüche 1 bis 3.

18. Verwendung des Präparates nach einem der Ansprüche 1 bis 3 zur Herstellung eines Mittels zur Behandlung von chronischen und akuten Yersinien-Infektionen.

19. Mittel zur Bekämpfung von Protozoen, bestehend aus einem Präparat nach einem der Ansprüche 1 bis 3.

20. Mittel zur Bekämpfung von Protozoen, enthaltend ein Präparat nach einem der Ansprüche 1 bis 3.

21. Verwnedung des Präparates nach einem der Ansprüche 1 bis 3 zur Herstellung eines Mittels zur Bekämpfung von Protozoen.

## Claims

1. Immunoglobulin preparation from bovine colostrum from the first 30, preferably the first 10 hours post partum, in which the colostrum is diluted with distilled water and then pasteurised, and the fat is separated and the casein precipitated and separated, obtainable by spray-drying of the colostral whey thus obtained at an input temperature of 145°C and an output temperature of 65°C.

2. Preparation according to claim 1, characterised in that the colostral whey is condensed before spray-drying.

3. Preparation according to either of claims 1 or 2, characterised in that before spray-drying it is subjected to treatment with caprylic acid.

4. Agent for treating diarrhoea in the case of AIDS or other conditions involving immunological deficiencies, consisting of a preparation according to one of claims 1 to 3.

5. Agent for treating diarrhoea in the case of AIDS or other conditions involving immunological deficiencies, containing a preparation according to one of claims 1 to 3.

6. Use of the preparation according to one of claims 1 to 3 for manufacturing an agent for combating diarrhoea in the case of AIDS or other conditions involving immunological deficiencies.

7. Agent for treating travel diarrhoea, consisting of a preparation according to one of claims 1 to 3.

8. Agent for treating travel diarrhoea, containing a preparation according to one of claims 1 to 3.

9. Use of a preparation according to one of claims 1 to 3 for manufacturing an agent for treating travel diarrhoea.

10. Agent for treating toxin-related infant diarrhoea, consisting of a preparation according to one of claims 1 to 3.

11. Agent for treating toxin-related infant diarrhoea, containing a preparation according to one of claims 1 to 3.

12. Use of the preparation according to one of claims 1 to 3 for manufacturing an agent for treating toxin-related infant diarrhoea.

13. Agent for treating stomach and intestinal ulcers and non-ulcerous forms of dyspepsia, consisting of a preparation according to one of claims 1 to 3.

14. Agent for treating stomach and intestinal ulcers and non-ulcerous forms of dyspepsia, containing a preparation according to one of claims 1 to 3.

15. Use of the preparation according to one of claims 1 to 3 for manufacturing an agent for treating stomach and intestinal ulcers and non-ulcerous forms of dyspepsia.

16. Agent for treating chronic and acute Yersinia infections, consisting of a preparation according to one of claims 1 to 3.

17. Agent for treating chronic and acute Yersinia infections, containing a preparation according to one of claims 1 to 3.

18. Use of the preparation according to one of claims 1 to 3 for manufacturing an agent for treating chronic and acute Yersinia infections.

19. Agent for combating protozoa, consisting of a preparation according to one of claims 1 to 3.

20. Agent for combating protozoa, containing a preparation according to one of claims 1 to 3.

21. Use of the preparation according to one of claims 1 to 3 for manufacturing an agent for combating protozoa.

## Revendications

1. Préparation immunoglobulinique de colostrum de bovin des 30 premières, de préférence des 10 premières heures post partum, le lait colostral étant dilué avec de l'eau distillée puis pasteurisé, les graisses séparées et la caséine précipitée et séparée, obtenue par séchage par pulvérisation du petit-lait colostral ainsi obtenu à une température initiale de 145°C et une température terminale de 65°C.

2. Préparation selon la revendication 1, caractérisée en ce que le petit-lait colostral est concentré avant le séchage par pulvérisation.

3. Préparation selon l'une des revendications 1 ou 2, caractérisée en ce qu'elle est soumise à un traitement par l'acide caprylique avant le séchage par pulvérisation.

4. Agent de traitement de la diarrhée dans le SIDA ou d'autres états déficitaires immunologiques, constitué par une préparation selon l'une des revendiacations 1 à 3.

5. Agent de traitement de la diarrhée dans le SIDA ou d'autres états déficitaires immunologiques, contenant une préparation selon l'une des revendications 1 à 3.

6. Usage de la préparation selon l'une des revendications 1 à 3 pour la production d'un agent de lutte contre la diarrhée dans le SIDA ou d'autres états déficitaires immunologiques.

7. Agent de traitement de la diarrhée des voyageurs, constitué par une préparation selon l'une des revendications 1 à 3.

8. Agent de traitement de la diarrhée des voyageurs, contenant une préparation selon l'une des revendications 1 à 3.

9. Usage d'une préparation selon l'une des revendications 1 à 3 pour la production d'un agent de traitement de la diarrhée des voyageurs.

10. Agent de traitement de la diarrhée infantile d'origine toxinique, constitué par une préparation selon l'une des revendications 1 à 3.

11. Agent de traitement de la diarrhée infantile d'origine toxinique, contenant une préparation selon l'une des revendications 1 à 3.

12. Usage de la préparation selon l'une des revendications 1 à 3 pour la production d'un agent de traitement de la diarrhée infantile d'origine toxinique.

13. Agent de traitement des ulcères gastro-duodénaux ainsi que des dyspepsies non ulcéreuses, constitué par une préparation selon l'une des revendications 1 à 3.

14. Agent de traitement des ulcères gastro-duodénaux ainsi que des dyspepsies non ulcéreuses, contenant une préparation selon l'une des revendica-tions 1 à 3.

15. Usage de la préparation selon l'une des revendications 1 à 3, pour la production d'un agent de traitement des ulcères gastro-duodénaux ainsi que des dyspepsies non ulcéreuses.

16. Agent de traitement des infections chroniques et aiguës à Yersinia, constitué par une préparation selon l'une des revendications 1 à 3.

17. Agent de traitement des infections chroniques et aiguës à Yersinia, contenant une préparation selon l'une des revendications 1 à 3.

18. Usage de la préparation selon l'une des revendications 1 à 3 pour la production d'un agent de traitement des infections chroniques et aiguës à Yersinia.

19. Agent de lutte contre les Protozoaires, constitué par une préparation selon l'une des revendications 1 à 3.

20. Agent de lutte contre les Protozoaires, contenant une préparation selon l'une des revendications 1 à 3.

21. Usage de la préparation selon l'une des revendications 1 à 3 pour la production d'un agent de lutte contre les Protozoaires.
